Europäisches Patentamt

(19) European Patent Office

Office européen des brevets

(11) Numéro de publication : **0 029 793**

**B1**

(12) **FASCICULE DE BREVET EUROPÉEN**

(45) Date de publication du fascicule du brevet :
24.08.83

(21) Numéro de dépôt : **80420121.8**

(22) Date de dépôt : **07.11.80**

(51) Int. Cl.³ : **A 61 M   1/03**

㉔ Appareiilage pour le contrôie des séances d'hémodialyse.

(30) Priorité : **23.11.79 FR 7929605**

(43) Date de publication de la demande :
**03.06.81 Bulletin 81/22**

(45) Mention de la délivrance du brevet :
**24.08.83 Bulletin 83/34**

(84) Etats contractants désignés :
**AT BE CH DE GB IT LI NL SE**

(56) Documents cités :
**BE A 877 159**
**FR A 1 449 713**
**FR A 2 053 869**
**FR A 2 179 980**
**FR A 2 264 335**
**GB A 2 003 274**

(73) Titulaire : **Thomasset, Auguste Louis**
**11, Rue Emile Zola**
**F-69002 Lyon (FR)**

(72) Inventeur : **Thomasset, Auguste Louis**
**11, Rue Emile Zola**
**F-69002 Lyon (FR)**

(74) Mandataire : **Monnier, Joseph et al**
**Cabinet Monnier 142-150, Cours Lafayette**
**F-69003 Lyon (FR)**

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

Jouve, 18, rue St-Denis, 75001 Paris, France

## Appareillage pour le contrôle des séances d'hémodialyse

On sait qu'en cas d'insuffisance ou de défaillance de la fonction rénale, on doit avoir périodiquement recours à des séances d'hémodialyse destinées à assurer, par épuration du sang, l'élimination des déchets provenant du métabolisme cellulaire. A cet effet la circulation sanguine du patient est momentanément déviée par un circuit extra-corporel pour traverser un rein artificiel qui a ainsi la charge de retirer de l'organisme l'eau en excès et les substances toxiques ou nocives qui se trouvent à l'état dissous dans les liquides interstitiels et intra-cellulaires.

Il va de soi que la quantité des déchets à éliminer varie de manière considérable en fonction de facteurs divers tenant notamment au poids et à la taille du patient, à son genre de vie, à la nature de son alimentation, etc... Le médecin néphrologue doit donc calculer le poids idéal à retirer de l'organisme de son patient au cours de chaque séance d'hémodialyse et cette détermination se fait à l'heure actuelle de manière empirique, à la lumière de phénomènes chimiques ou physiologiques antérieurement relevés sur le patient. Une fois cette opération effectuée le praticien joue sur la durée de la séance et sur le débit horaire de la circulation du sang à travers le rein artificiel.

On comprend cependant que par suite du mode même de la détermination du poids idéal à retirer, il y a lieu de procéder à une surveillance sérieuse du déroulement de chaque séance. Ce sont normalement la pression artérielle et ses modifications au cours de l'opération qui devraient fournir les informations les plus valables ; toutefois l'expérience démontre que des accidents graves surviennent du fait que la tension artérielle ne peut être suivie en continu et que des détections périodiques ne permettent pas d'obtenir une image fidèle des effets successifs de l'hémodialyse sur l'organisme du patient.

Il est aussi connu de procéder à une surveillance en continue avec système d'alarme, en contrôlant sur un circuit extra corporel le sang circulant dans une éprouvette grâce à des impulsions haute-fréquence émises à travers cette éprouvette. (FR-A-2 053 869).

On rappellera que les accidents rencontrés en cours d'hémodialyses sont en pratique de deux ordres différents :

1. Certains surviennent au niveau du système circulatoire et sont dus au volume d'eau retiré du plasma, ce volume étant trop important par suite d'une vitesse de retrait trop élevée ou d'une durée de séance trop prolongée.

2. D'autres sont consécutifs au retrait non mesuré d'éléments chimiques dont la présence dans l'organisme en une quantité bien déterminée est indispensable à l'équilibre vital (homéostasie). Ils se répercutent à la fois au niveau du sang et au niveau des tissus de l'ensemble corporel.

C'est à la détection systématique de ces deux risques d'accidents et à leur compensation automatique qu'entend s'attacher la présente invention, et ce à l'aide d'un appareillage dont l'intervention s'exerce à partir du circuit sanguin extra-corporel.

L'invention se fonde en premier lieu sur la mesure en continu, tout au long de la séance d'hémodialyse, des variations éventuelles de l'hématocrite, c'est-à-dire du volume ou poids relatif des globules du sang par rapport au plasma.

On conçoit sans peine que ce rapport puisse augmenter au cours de la séance d'hémodialyse du fait du retrait progressif de liquide ; la quantité relative de globules risque de devenir trop importante si l'on n'y veille. Or la mesure du plasma et de l'hématocrite est susceptible d'être réalisée en continu en relevant l'impédance à basse fréquence (de l'ordre de 5 kHz) d'un volume constant de sang en circulation, cette impédance augmentant si la partie globulaire bien que restant fixe prend une place plus importante dans le volume considéré.

Par ailleurs l'invention tire parti des modifications qui interviennent au cours de l'hémodialyse dans la répartition ionique au niveau du plasma et du liquide interstitiel d'une part, au niveau des cellules d'autre part.

On a pu remarquer que si les échanges ioniques avaient tout d'abord lieu entre le plasma et le rein artificiel à travers la membrane de celui-ci, la modification engendrée au niveau du plasma retentissait très rapidement sur le liquide interstitiel à travers la paroi des capillaires, puis sur le liquide intra-cellulaire à travers la membrane cellulaire. Dans ces conditions on comprend finalement qu'il existe une liaison quantitative entre la concentration du sang en ions et le volume du liquide intra-cellulaire. Or les travaux antérieurs du Demandeur ont démontré que la détermination du volume de liquide intra-cellulaire était susceptible d'être obtenue par mesure de l'impédance.

Compte tenu des remarques qui précèdent, l'appareillage de contrôle suivant la présente invention, du genre comprenant un circuit d'alarme agencé de manière à fonctionner sous l'effet d'un signal émis par un appareil de mesure lorsque la variable à surveiller, captée sur le circuit extra-corporel associé au rein artificiel de traitement, dépasse un seuil prédéterminé, est caractérisé en ce que l'appareil de mesure est un impédancemètre susceptible d'être sélectivement relié soit à un premier jeu d'électrodes monté sur une cellule à volume constant placée sur le circuit extra-corporel en amont du rein artificiel, soit à un second jeu d'électrodes propre à être fixé sur le corps du patient, lequel impédancemètre, du type en soi connu comportant un circuit basse fréquence à environ 5 kHz et un circuit haute fréquence à environ 1 MHz, est associé à un comparateur de déclenchement lui-même placé sous la dépendance d'un appareil pour l'affichage de la variation Δz (à 5 kHz) de l'impédance du sang qui traverse la cellule précitée.

Conformément à un mode de mise en œuvre préféré de l'invention, le signal émis par le comparateur provoque, en plus de l'actionnement du circuit d'alarme, la diminution immédiate du débit de circulation dans le rein artificiel et le déclenchement retardé d'un dispositif d'injection thérapeutique programmé qui assure de manière automatique l'introduction dans l'organisme d'une quantité déterminée d'une substance (en pratique un sérum à base de chlorure de sodium) propre à favoriser le transfert du liquide interstitiel dans le système vasculaire à travers la barrière capillaire.

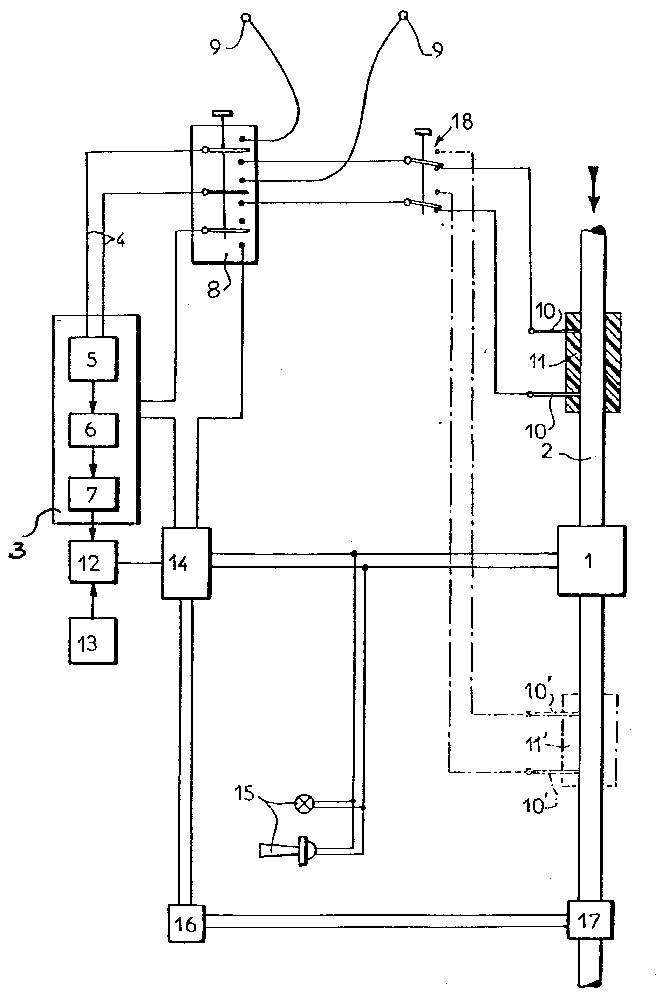

Le dessin annexé, donné à titre d'exemple, permettra de mieux comprendre l'invention, les caractéristiques qu'elle présente et les avantages qu'elle est susceptible de procurer :

La figure unique de ce dessin est un schéma montrant l'agencement général d'un appareillage de contrôle établi conformément à l'invention.

Sur ce schéma la référence 1 désigne un rein artificiel de construction classique équipé d'une pompe à dépression ou organe similaire propre à assurer la circulation du sang dans un circuit extra-corporel 2 dont les extrémités sont reliées au système vasculaire du patient.

L'appareillage de contrôle et d'intervention suivant l'invention comprend en premier lieu un impédancemètre 3, préférablement du type de celui qui fait l'objet du brevet français N° 1 344 459 déposé le 18 Octobre 1962 au nom du Demandeur. On rappellera simplement qu'un tel appareil, du type fonctionnant à plusieurs fréquences, permet la mesure de la résistance opposée par les tissus organiques au passage de courants alternatifs en basse fréquence à 5 kHz et à haute fréquence à 1 MHz entre deux électrodes placées l'une sur le dos d'une main, l'autre sur le dos du pied contro-latéral (méthode dite de Thomasset). L'entrée 4 de l'impédancemètre aboutit à un système d'oscillateurs 5 lié à un voltmètre 6 lui-même associé à un logiciel 7 à affichage numérique.

Conformément à l'invention, sur l'entrée 4 est branché un commutateur 8 qui permet de relier sélectivement l'impédancemètre 3 à l'un ou à l'autre de deux jeux d'électrodes 9 et 10. Le jeu d'électrodes 9 est agencé de manière à être utilisé à la façon ci-dessus exposée (méthode de Thomasset), tandis que les deux électrodes 10 sont implantées dans un bloc ou cellule 11, préférablement réalisé en matière synthétique transparente.

Ce bloc 11 est disposé sur le circuit 2 en amont du rein artificiel 1 ; les électrodes 10 définissant dans le bloc 11 un volume déterminé de manière parfaitement précise, l'impédancemètre 3 est ainsi susceptible d'indiquer, à partir de la mesure de l'impédance, la quantité de plasma présente dans le sang qui parcourt le circuit 2.

A l'impédancemètre 3 est associé un circuit comparateur 12 qui reçoit simultanément les informations du logiciel 7 et celles d'un appareil de présélection 13, ce dernier permettant par affichage de déterminer le seuil de déclenchement pour l'émission d'un signal. Ce signal est recueilli par un relais 14 qui commande un circuit d'alarme 15 du type sonore et/ou visuel et qui est relié à la pompe ou autre organe qui assure dans le rein artificiel 1 la circulation du sang dans le circuit 2.

On notera que le relais 14 est alimenté à travers un contact supplémentaire du commutateur 8, si bien qu'il ne peut fonctionner que lorsque l'impédancemètre 3 est relié aux électrodes 10. Par ailleurs ce relais 14 alimente, à travers un mécanisme de temporisation 16, un dispositif d'injection automatique 17, placé sur le circuit 2 en aval du rein 1.

Le fonctionnement et l'utilisation de l'appareillage suivant l'invention découlent des explications qui précèdent.

Après branchement du circuit 2 sur le patient et pose des électrodes 9 entre main et pied controlatéral, le praticien manœuvre le commutateur 8 de façon à ce que l'impédancemètre 3 soit relié aux électrodes 10 de la cellule 11. Puis il affiche sur l'appareil de présélection 13 la variation autorisée Δz (à 5 kHz) de l'impédance du sang circulant dans la cellule 11 précitée ; ce Δz (à 5 Hz) représente en fait un pourcentage de l'impédance actuelle du sang telle que relevée au début de l'opération. Bien entendu ce pourcentage est susceptible de varier d'un sujet à un autre suivant l'hématocrite, et éventuellement chez le même sujet d'une séance à une autre ; cette valeur doit en conséquence être ajustée à chaque fois.

Ceci fait le néphrologue n'a plus à assurer une surveillance continue du patient puisqu'il sait que le système d'alarme 15 l'avertira dès que la quantité d'eau retirée du sang par le rein artificiel 1 atteindra la valeur correspondant à Δz (à 5 kHz) telle qu'affichée sur l'appareil de présélection 13. A ce moment en effet le relais 14 qui reçoit le signal émis par le circuit comparateur 12 provoque, non seulement l'actionnement de l'alarme 15, mais également l'arrêt ou tout au moins la diminution sensible du débit de la pompe ou organe similaire du rein 1 au cours de la phase d'ultra-filtration. De plus, dans le laps de temps (de l'ordre de 2 à 3 minutes) introduit par le mécanisme temporisateur 16, le dispositif 17 est actionné de manière automatique.

Le praticien règle alors l'impédancemètre 3 de façon à ce que celui-ci débite un courant d'environ 1 MHz et il manœuvre le commutateur 8 de façon à ce que ledit courant soit envoyé dans le jeu d'électrodes 9. Dès ce moment le relais 14 est mis hors service et l'on doit suivre sur l'affichage du logiciel 7 le résultat de l'injection de sérum opérée par le dispositif 17. Si après un cours temps l'impédancemètre ne permet pas de déceler, par la mesure de l'impédance dans l'ensemble de l'organisme du patient, la présence d'une quantité de liquide interstitiel suffisante, la séance doit être arrêtée. Si au contraire l'impédance relevée est satisfaisante, on peut poursuivre l'élimination des substances dissoutes et notamment les ions Na+ et K+, jusqu'à atteindre la valeur souhaitable.

En définitive, de par l'action de surveillance constante qu'il exerce tout au long des séances d'hémodialyse, l'appareillage suivant l'invention permet d'éviter de façon efficace les deux principaux risques d'accidents rencontrés en pratique, à savoir le choc volumique provoqué par un retrait d'une trop grande quantité d'eau, et le choc osmotique engendré par la disparition d'une quantité trop importante d'éléments chimiques.

En vue d'améliorer la capacité des informations susceptibles d'être fournies par l'appareillage suivant l'invention, on peut avantageusement faire comporter à celui-ci un second bloc ou cellule 11' représenté en tracé interrompu sur le dessin ; cette cellule 11' est identique à la première 11, mais elle est placée sur le circuit 2 en aval du rein artificiel 1. Ses deux électrodes 10' sont reliées à un commutateur 18 prévu sur le circuit reliant les électrodes 10 au commutateur 8, si bien qu'au cours de la seconde phase du processus ci-dessus exposé, la manœuvre de ce commutateur 18 permet à tout moment au praticien de vérifier le fonctionnement du rein artificiel 1 par comparaison sur l'impédancemètre 3 des informations reçues des électrodes 10 et 10'.

**Revendications**

1. Appareillage pour le contrôle des séances d'hémodialyse, du genre comprenant un circuit d'alarme (15), agencé de manière à fonctionner sous l'effet d'un signal émis par un appareil de mesure lorsque la variable à surveiller, captée sur le circuit extra-corporel associé au rein artificiel de traitement, dépasse un seuil prédéterminé, caractérisé en ce que l'appareil de mesure est un impédancemètre (3) susceptible d'être sélectivement relié soit à un premier jeu d'électrodes (10) monté sur une cellule (11) à volume constant placée sur le circuit extra-corporel (2) en amont du rein artificiel (1), soit à un second jeu d'électrodes (9) propre à être fixé sur le corps du patient, lequel impédancemètre, du type en soi connu comportant un circuit basse fréquence à 5 kHz et un circuit haute fréquence à 1 MHz, est associé à un comparateur de déclenchement (12) pour l'émission d'un signal lui-même placé sous la dépendance d'un appareil (13) sur lequel a été affichée la variation autorisée Δz (à 5 kHz) de l'impédance du sang qui traverse la cellule (11) précitée, variation qui constitue le seuil prédéterminé.

2. Appareillage suivant la revendication 1, caractérisé en ce que la cellule (11) est constituée par un bloc en matière isolante, creusé d'un canal axial de circulation sur la paroi duquel affleurent les deux électrodes (10) orientées de manière substantiellement transversale.

3. Appareillage suivant l'une quelconque des revendications 1 et 2, caractérisé en ce que le second jeu d'électrodes (9) est agencé pour être branché, à la façon en soi connue, entre la main et le pied controlatéral du patient.

4. Appareillage suivant l'une quelconque des revendications 1 à 3, caractérisé en ce que le circuit d'alarme (15) est relié, à travers un mécanisme temporisateur (16), à un dispositif (17) pour l'injection automatique d'une solution dans le circuit extra-corporel (2), en aval du rein artificiel (1).

5. Appareillage suivant l'une quelconque des revendications 1 à 4, du genre dans lequel on agit sur la vitesse et/ou le débit de la circulation du sang à l'intérieur du rein artificiel (1), caractérisé en ce que sur le circuit d'alarme (15) est directement branché un circuit électrique relié à la pompe du rein artificiel (1).

6. Appareillage suivant l'une quelconque des revendications 1 à 5, caractérisé en ce qu'il comprend un jeu supplémentaire d'électrodes (10') monté sur une seconde cellule (11') placée sur le circuit extra-corporel (2) en aval du rein artificiel (1), ce jeu étant susceptible d'être relié à l'impédancemètre (3) à la place du premier jeu d'électrodes (10).

**Claims**

1. An apparatus for monitoring haemodialysis sessions, of the kind comprising an alarm circuit (15), arranged in such a way as to operate under the effect of a signal sent out by a measuring circuit when the variable to be monitored, read from the circuit exterior to the body connected to an artificial kidney, exceeds a predetermined threshold, characterised in that the measuring apparatus is an impedance meter (3) able to be connected selectively either to a first set of electrodes (10) mounted on a cell (11) of constant volume placed in the external circuit (2) before an artificial kidney (1), or to a second set of electrodes (9) able to be fixed on the body of the patient, the impedance meter — of a type itself known, comprising a low frequency circuit at 5 kHz and a high frequency circuit at 1 MHz — being connected to a trigger-comparator (12) for emitting a signal which is itself dependent on an apparatus (13) which has been set at the authorised variation Δz (at 5kHz) of the impedance of the blood which is passing through the above-mentioned cell (11) this variation constituting the predetermined threshold.

2. An apparatus according to claim 1, characterised in that the cell (11) is constituted by a block of insulating material drilled with an axial circulation channel in the wall of which are set two flush electrodes (10) which lie closely transversal to the block.

3. An apparatus according to either one of claims 1 and 2, characterised in that the second set of electrodes (9) is designed in the way itself known between the hand and the controlateral foot of the patient.

4. An apparatus according to any one of claims 1 to 3, characterised in that the alarm circuit (15) is connected, through a timing mechanism (16) to a device (17) for the automatic injection of a solution into the external circuit (2) after the artificial kidney (1).

5. An apparatus according to any one of the claims 1 to 4, of the kind in which the speed and/or the volume of the circulation of the blood within the artificial kidney is controlled, characterised in that the alarm circuit (15) is directly connected to an electrical circuit connected to the pump of the artificial kidney (1).

6. An apparatus according to any one of claims 1 to 5, characterised in that it comprises an additional set of electrodes (10') fitted to a second cell (11') placed in the external circuit (2) after the artificial kidney (1), this set being able to be connected to the impedence meter (3) instead of the first set of electrodes (10).

**Ansprüche**

1. Einrichtung zur Überwachung von Dialysebehandlungen mit einem Überwachungsstromkreislauf (15) der auf ein von einem Meßgerät abgegebenes Signal anspricht, wenn die zu überwachende, veränderliche Größe, die in dem an die zur Behandlung verwendete künstliche Niere angeschlossenen, außerkörperlichen Kreislauf abgenommen wird, eine zulässige Ansprechschwelle überschreitet, dadurch gekennzeichnet, daß als Meßgerät ein Scheinwiderstandsmeßgerät (3) vorgesehen ist, das an einen ersten Elektrodensatz (10), angeordnet in einer Meßzelle (11) konstanten Volumens in dem außerkörperlichen Kreislauf (2) vor der künstlichen Niere (1), oder an einen zweiten Elektrodensatz (9), befestigt am Körper des Patienten, anschließbar ist, wobei der an sich bekannte Scheinwiderstandsmesser einen Niederfrequenzmeßkreis von 5 kHz und einen Hochfrequenzmeßkreis von 1 MHz aufweist und an einem das Ausgangssignal angebenden Auslösekomperator (12) angeschlossen ist, der seinerseits in Abhängigkeit von einem Referenzgeber (13) angesteuert ist, welchem die zugelassene Veränderung Δz (bei 5 kHz) der Impedanz des Blutes, das die Meßzelle (11) durchfließt, als die vorgegebene Ansprechschwelle eingegeben ist.

2. Einrichtung nach Anspruch 1, dadurch gekennzeichnet, daß die Meßzelle (11) aus einem Block aus Isoliermaterial besteht, in welchem ein axialer Umlaufkanal gebohrt ist, auf dessen Wandungen die beiden Elektroden (10) satt anliegen und im wesentlichen quer orientiert sind.

3. Einrichtung nach einem der Ansprüche 1 und 2, dadurch gekennzeichnet, daß der mit dem Körper des Patienten zu verbindende Elektrodensatz (9) in an sich bekannter Weise kontralaterial zwischen der Hand und dem Fuß des Patienten angebracht ist.

4. Einrichtung nach einem der Ansprüche 1 bis 3, dadurch gekennzeichnet, daß ein Überwachungsstromkreis (15) über ein Verzögerungsglied (16) mit einer Vorrichtung (17) zur automatischen Injektion einer Lösung in den außerkörperlichen Kreislauf (2) verbunden ist, wobei die Vorrichtung (17) zur automatischen injektion abströmseitig der künstlichen Niere (1) angeordnet ist.

5. Einrichtung nach einem der Ansprüche 1-4, derart, daß man auf die Geschwindigkeit und/ oder auf den Blutkreislaufdurchfluß im Inneren der künstlichen Niere (1) einwirken kann, daß der Überwachungsstromkreis (15) direkt an einen mit der Pumpe der künstlichen Niere (1) verbundenen Stromkreislauf angeschlossen ist.

6. Einrichtung nach einem der Ansprüche 1-5, dadurch gekennzeichnet, daß ein zusätzlicher Elektrodensatz (10') vorgesehen ist, der in einer zweiten Meßzelle (11') angeordnet ist, die in dem außerkörperlichen Kreislauf (2) unterhalb der künstlichen Niere (1) sitzt, wobei dieser Satz mit dem Scheinwiderstandsmesser (3) an Stelle des ersten Elektrodensatzes (10) verbunden werden kann.

0 029 793